Europäisches Patentamt
European Patent Office
Office européen des brevets

(19) (11) Publication number: **0 161 350 A1**

(12) # EUROPEAN PATENT APPLICATION

(21) Application number: **84116509.5**

(22) Date of filing: **29.11.82**

(51) Int. Cl.⁴: **C 07 D 223/16**, C 07 C 87/28 // C07C103/78, C07C91/06

(30) Priority: **27.11.81 US 325249**
**14.07.82 US 398015**
**08.10.82 EP 82305361**

(43) Date of publication of application: **21.11.85 Bulletin 85/47**

(84) Designated Contracting States: **AT BE CH DE FR GB IT LI LU NL SE**

(60) Publication number of the earlier application in accordance with Art. 76 EPC: **0080779**

(71) Applicant: **SMITHKLINE BECKMAN CORPORATION, P.O. Box 7929 1 Franklin Plaza, Philadelphia Pennsylvania 19101 (US)**

(72) Inventor: **DeMarinis, Robert Michael, 104 Cedarbrook Road, Ardmore Pennsylvania 19003 (US)**
Inventor: **Hieble, Jacob Paul, 2107 Mount Vernon Street, Philadelphia Pennsylvania 19130 (US)**
Inventor: **Matthews, William David, 812 Happy Creek Lane, West Chester Pennsylvania 19380 (US)**

(74) Representative: **Johnston, Walter Paul et al, Patent Department SMITH KLINE & FRENCH LABORATORIES LTD Mundells, Welwyn Garden City Hertfordshire AL7 1EY (GB)**

(54) **Process for preparing benzazepines.**

(57) The invention provides a process for preparing a compound of formula (I) and pharmaceutically acceptable salts thereof

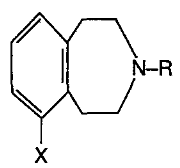

(I)

where R is lower alkyl of from 1 to 3 carbon atoms or allyl and

X is halogen which comprises cyclising a compound of formula (II):

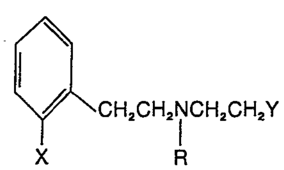

(II)

where R and X are as defined with reference to formula (I) and Y is halogen under Friedel Crafts conditions.

EP 0 161 350 A1

# A PROCESS FOR PREPARING BENZAZEPINES

This invention relates to a process for preparing N-substituted 2,3,4,5-tetrahydro-1H-3-benzazepines and intermediates for use in this process.

In our co-pending European Patent Specification No. 0080779 we have disclosed certain benzazepine compounds that are useful as alpha2 antagonists.

The compounds concerned are compounds of formula (I):-

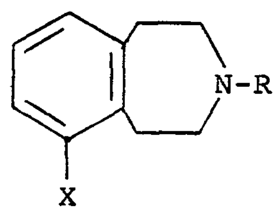

(I)

and their pharmaceutically acceptable salts in which

R is lower alkyl of from 1 to 3 carbon atoms or allyl;

and

X is halogen such as chloro, bromo or fluoro.

A particularly preferred compound of formula (I) is one where R is methyl and X is chloro, that is 6-chloro-2,3,4,5-tetrahydro-3-methyl-1H-3-benzazepine.

According to the present invention compounds of formula (I) are prepared by cyclising a compound of formula (II):-

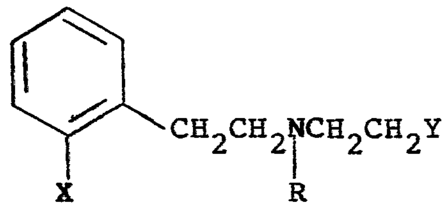

(II)

where X and R are as previously defined and Y is halogen under Friedel Crafts conditions in the presence of a Lewis acid and thereafter optionally converting the compound of formula (I) so obtained into a pharmaceutically acceptable salt.

The cyclisation step is carried out using a Lewis acid for example aluminium chloride, aluminium bromide, titanium chloride or antimony chloride.

Preferably the catalyst is aluminium chloride.

Preferably the reaction is carried out in a melt of aluminium chloride and ammonium chloride at elevated temperature.

The compounds of formula (II) are novel and form a further aspect of the invention.

Accordingly, in a further aspect the invention provides a compound of formula (II) as previously defined.

Preferably Y is chlorine or bromine, especially chlorine.

Compounds of formula (II) can in turn be prepared by reacting a compound of formula (III):-

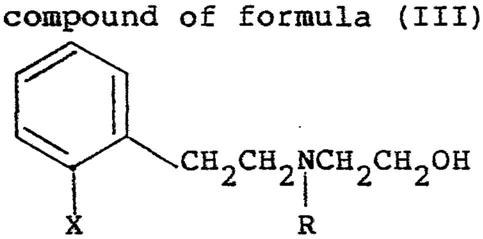

(III)

where X and R are as previously defined with a halogenating agent.

Preferably the halogenating agent is phosphorous pentachloride.

The above compounds of formula (III) can be prepared as follows:-

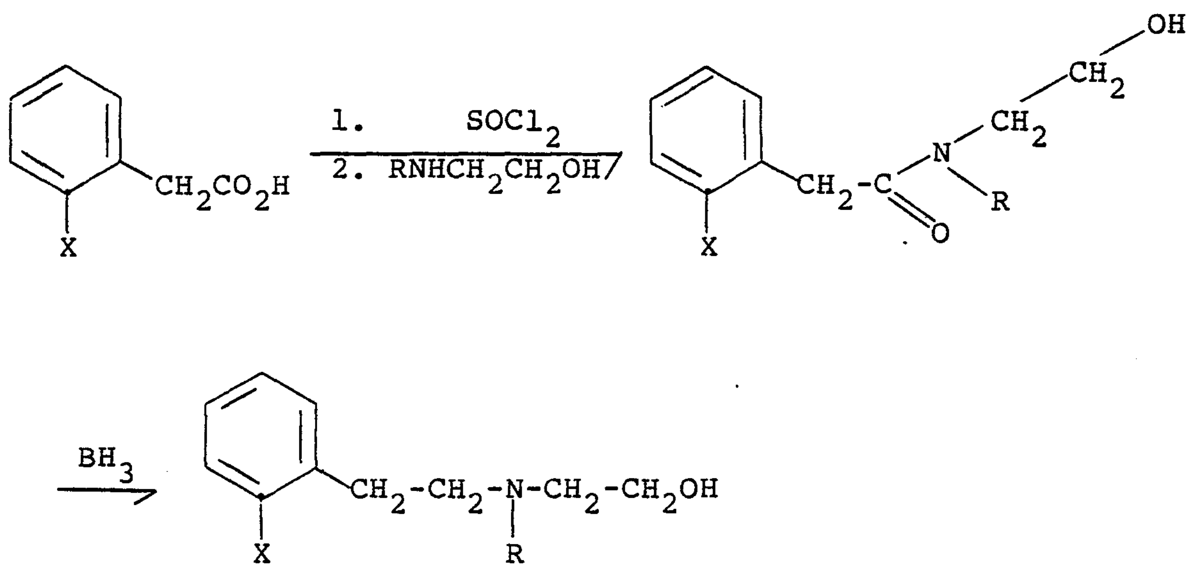


The terms X and R are as defined above.

According to the above procedure, a halophenyl acetic acid is treated with thionyl chloride followed by an appropriate amino alcohol. The resultant amide is reduced by any well known agent such as, for example, borane.

The pharmaceutically acceptable acid addition salts of compounds of formula (I) can be prepared by methods well known to the art from both inorganic or organic acids, for example: maleic, fumaric, benzoic, ascorbic, pamoic, succinic, bis-methylenesalicylic, methanesulfonic, ethanedisulfonic, acetic, oxalic, propionic, tartaric,

salicylic, citric, gluconic, aspartic, stearic, palmitic, itaconic, glycolic, p-aminobenzoic, glutamic, benzenesulfonic, hydrochloric, hydrobromic, sulfuric, cyclohexylsulfamic, phosphoric and nitric acids.

The following Examples illustrate the invention. The temperatures are in degrees Centigrade.

Example 1

A mixture of 125 g. (0.73 mol) of O-chlorophenylacetic acid, 155 g. (1.3 mol) of thionyl chloride and 2-3 drops of dimethylformamide in 1500 ml. of toluene was stirred at room temperature for three hours. The toluene was evaporated under reduced pressure to give an oil which was dissolved in 200 ml. of methylene chloride. This was added dropwise to a solution of 165 g. (2.2 mol) of N-methylamino ethanol in 1 liter of methylene chloride. After addition was complete, the solution was stirred at room temperature for three hours. The organic solution was washed with water, dilute hydrochloric acid and saturated sodium chloride, dried over magnesium sulfate, filtered and evaporated to give 2-chloro-N-(2-hydroxyethyl)-N-methylbenzeneacetamide as a crystalline solid, m.p. 77°.

To 400 ml. of a 1 mol solution of borane in tetrahydrofuran was added dropwise a solution of 43 g. of the above amide in 350 ml. of tetrahydrofuran at a rate sufficient to maintain a gentle reflux. After addition was complete, the solution was refluxed for two hours, cooled in an ice bath and treated carefully with dilute hydrochloric acid to destroy excess borane. The majority of the solvent was removed under vacuum and the residue heated on a steam bath for one hour. The mixture was diluted with 300 ml. of water and extracted with ether. The aqueous layer was made basic with 40% sodium hydroxide and extracted with ether. The combined basic extracts were washed with water and saturated sodium chloride, dried and evaporated to give 2-[[2-(2-chlorophenyl)ethyl]methylamino]ethanol.

A suspension of 36 g. (0.173 mol) of phosphorous pentachloride in 300 ml. of methylene chloride was treated dropwise with a solution of 37 g. (0.173 mol) of the 2-[[2-(2-chlorophenyl)ethyl]methylamino]ethanol in 150 ml. of methylene chloride. After addition was complete, the mixture was refluxed overnight, evaporated to dryness and partitioned between dilute hydrochloric acid and ether. The aqueous layer was made basic with 10% sodium hydroxide and extracted well with ether. The ether extracts were washed with water and saturated sodium chloride, dried over magnesium sulfate and filtered. Addition of a saturated solution of ethereal hydrogen chloride gave a solid precipitate which was removed by filtration, washed with ether and dried to give 2-chloro-N-(2-chloroethyl)-N-methylbenzene ethanamine hydrochloride, m.p. 110°.

To a mixture of 41.5 g (0.155 mol) of the above chloro ethanamine hydrochloride and 6.26 g. (0.117 mol) of ammonium chloride was added 41 g. of anhydrous aluminium chloride. The reaction became homogenous, melted and exothermed. It was placed in an oil bath which had been heated to 175° and stirred for thirty minutes. An additional 20 g. of aluminium chloride was added and the mixture heated for another thirty minutes. A final 41 g. portion of aluminium chloride was added and the reaction heated for twenty hours. It was cooled to 140° and poured into 3 l. of ice water containing 300 ml. of concentrated hydrochloric acid and stirred for fifteen minutes. Sixty grams of sodium potassium tartrate was added and stirred until solution was effected. It was made basic with 40% sodium hydroxide, extracted twice with ether and the combined extracts washed with water, and saturated sodium chloride, dried and reduced in volume by half. Addition of a solution of saturated ethereal hydrogen chloride

gave a solid precipitate which was collected, washed with ether and dried to give a white solid. Crystallization from methanol-ethyl acetate gave 6-chloro-3-methyl-2,3,4,5-tetrahydro-1H-3-benzazepine hydrochloride, m.p. 268-270°.

Example 2

Following the procedure of Example 1 and substituting 2-[[2-(2-chlorophenyl)ethyl]ethylamino]-ethanol and 2-[[2-(2-chlorophenyl)ethyl]allylamino]-ethanol for 2-[[2-(2-chlorophenyl)ethyl]methylamino]-ethanol yields the following respective products: 6-chloro-3-ethyl-2,3,4,5-tetrahydro-1H-3-benzazepine and 6-chloro-3-allyl-2,3,4,5-tetrahydro-1H-3-benzazepine.

<u>Claims</u>

1. A process for preparing a compound of formula (I) and pharmaceutically acceptable salts thereof

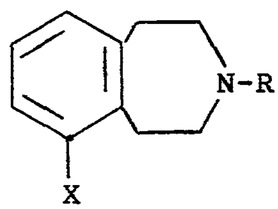

(I)

where R is lower alkyl of from 1 to 3 carbon atoms or allyl and
X is halogen which comprises cyclising a compound of formula (II):

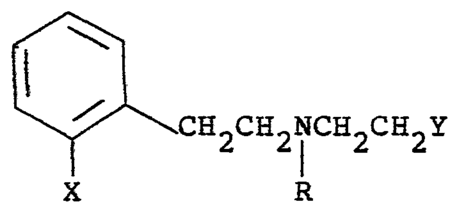

(II)

where R and X are as defined with reference to formula (I) and Y is halogen under Friedel Crafts conditions.

2. A process as claimed in claim 1 where the Lewis acid is aluminium chloride.

3. A process as claimed in claim 1 or claim 2 where Y is chlorine.

4. A process as claimed in claim 3 where the reaction is carried out in a melt of ammonium chloride.

5. A compound of formula (II):-

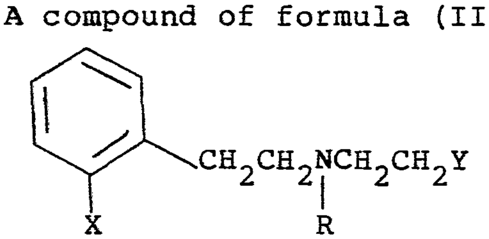

(II)

where X, R and Y are as defined in claim 1.

6. A compound as claimed in claim 5 where Y is chlorine.

7. A compound as claimed in claim 5 or claim 6 where X is chlorine and R is methyl.

8. A process for preparing a compound of formula (II) as defined in claim 5 which comprises reacting a compound of formula (III):-

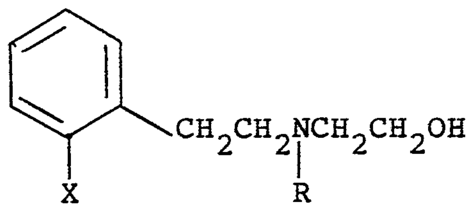

(III)

with a halogenating agent.

Claims for the designated state AT

1. A process for preparing a compound of formula (I) and pharmaceutically acceptable salts thereof

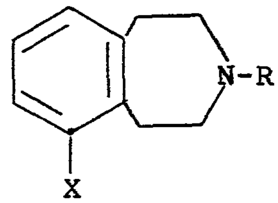

(I)

where R is lower alkyl of from 1 to 3 carbon atoms or allyl and
X is halogen which comprises cyclising a compound of formula (II):

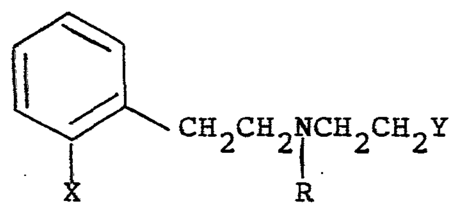

(II)

where R and X are as defined with reference to formula (I) and Y is halogen under Friedel Crafts conditions.

2. A process as claimed in claim 1 where the Lewis acid is aluminium chloride.

3. A process as claimed in claim 1 or claim 2 where Y is chlorine.

4. A process as claimed in claim 3 where the reaction is carried out in a melt of ammonium chloride.

5. A process for preparing a compound of formula (II) as defined in claim 5 which comprises reacting a compound of formula (III):-

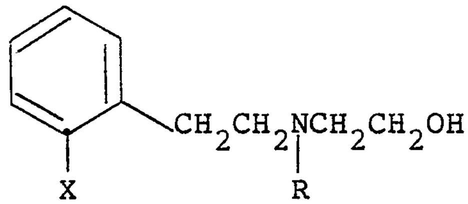

(III)

with a halogenating agent.

European Patent Office

# EUROPEAN SEARCH REPORT

0161350

Application number

EP 84 11 6509

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | GB-A-1 221 324 (J.R. GEIGY) * Whole document * | 1-4 | C 07 D 223/16<br>C 07 C 87/28 //<br>C 07 C 103/78<br>C 07 C 91/06 |
| X | EP-A-0 008 405 (SMITHKLINE) * Whole document * | 1-4 | |
| X | CHEMICAL ABSTRACTS SERVICE, REGISTRY HANDBOOK, Number Section 1965-1971, page 578R, Columbus, Ohio, US; * Page 578R, registry nos. 1208-26-0, 1208-27-1 and 1208-28-2 * | 5-7 | |
| | | | **TECHNICAL FIELDS SEARCHED (Int. Cl.4)** |
| | | | C 07 D 223/00<br>C 07 C 87/00 |

The present search report has been drawn up for all claims

| Place of search | Date of completion of the search | Examiner |
|---|---|---|
| THE HAGUE | 24-07-1985 | ALLARD M.S. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503. 03.82